# EUROPÄISCHE PATENTSCHRIFT

(11) **EP 0 941 054 B1**
(45) Veröffentlichungstag und Bekanntmachung des Hinweises auf die Patenterteilung: **19.06.2002**
(21) Anmeldenummer: 97910423.9
(22) Anmeldetag: 08.10.1997
(51) Int. Cl.: A61K 7/48, A61K 31/56

(54) **VERWENDUNG VON STEROLSULFATEN ALS WIRKSTOFFE ZUR HERSTELLUNG VON MITTELN ZUR INHIBIERUNG VON SERINPROTEASEN**
USE OF STEROLSULFATES AS ACTIVE SUBSTANCES FOR PRODUCING MEANS TO INHIBIT SERIN PROTEASES
UTILISATION DE STEROLSULFATES COMME SUBSTANCES ACTIVES POUR LA PRODUCTION DE MOYENS DESTINES A L'INHIBITION DES SERINES PROTEASES

(30) Priorität: 17.10.1996 DE 19642872
(43) Veröffentlichungstag der Anmeldung: 15.09.1999
(73) Patentinhaber: Cognis Deutschland GmbH & Co. KG, 40589 Düsseldorf (DE)
(72) Erfinder: WACHTER, Rolf, D-40595 Düsseldorf (DE); TESMANN, Holger, D-41363 Jüchen (DE); BEHLER, Ansgar, D-46240 Bottrop (DE); MAURER, Karl-Heinz, D-40699 Erkrath (DE)
(86) Internationale Anmeldenummer: EP9705524
(87) Internationale Veröffentlichungsnummer: WO9817244

(56) Entgegenhaltungen:
- EP-A- 0 723 775
- WO-A-88/01274
- WO-A-89/01327
- WO-A-90/01323
- PATENT ABSTRACTS OF JAPAN vol. 017, no. 361, 8.Juli 1993 & JP 05 051314 A (KANEBO), 2.März 1993,
- PATENT ABSTRACTS OF JAPAN vol. 09, no. 331 (C-321) [2054] , 25.Dezember 1985 & JP 60 161911 A (KANEBO), 23.August 1985,
- PATENT ABSTRACTS OF JAPAN vol. 11, no. 115 (C-415) [2562] , 10.April 1987 & JP 61 260004 A (KANEBO), 18.November 1986,

## Beschreibung

### Gebiet der Erfindung

Die Erfindung betrifft die Verwendung von Sterolsulfaten als Wirkstoffe zur Bekämpfung der Faltenbildung in der Haut sowie von Hauterkrankungen wie beispielsweise Schuppenflechte, Psoriasis oder UV-Erythemen.

### Stand der Technik

Die altersbedingte Faltenbildung wird durch den Abbau von verschiedenen Makromolekülen wie beispielsweise Elastin und Kollagen hervorgerufen, für den Elastasen im Stratum corneum verantwortlich sind. Auch eine Vielzahl von entzündlichen Hauterkrankungen, wie beispielsweise Schuppenflechte oder UV-Erytheme, lassen sich ursächlich mit einer erhöhten Elastasekonzentration in den oberen Hautschichten in Verbindung bringen [vgl. R.Voegeli et al in **Cosm.Toil. 111, 51 (1996)].**

Der Faltenbildung in der Haut wird in der Regel nicht durch physiologisch wirksame Prinzipien, sondern durch kosmetische Mittel entgegengewirkt. Viele sogenannte "Antiaging Produkte" enthalten mit Wasser oder wäßrigen Wirkstoffen beladene Liposomen, die durch die Fettschicht der Haut in die Epidermis gelangen, sich dort allmählich auflösen und durch die kontinuierliche Wasserabgabe die Hautvertiefungen füllen und den Feuchtigkeitsgehalt der Haut regulieren. Dieser Effekt stellt jedoch keine Bekämpfung der Ursachen dar, sondern hat lediglich einen sogenannten "repair effect", der zudem nur über eine kurze Zeit andauert.

Im Gegensatz zu dieser rein kosmetischen Anwendung dienen der Bekämpfung der Schuppenflechte beispielsweise cytostatische Wirkstoffe, wie etwa Selensulfid, Cadmiumsulfid, Zinkpyrithione oder Corticosteroide, deren medizinische Wirkung z.B. auf einer Reduzierung der Mitoseaktivität in der Basalmembran beruht. Wegen der bekannten Nebenwirkungen sollten diese Stoffe jedoch nicht über längere Zeiträume eingesetzt werden. Weiterhin läßt sich die Schuppenflechte durch antiseptische Wirkstoffe, wie beispielsweise Selenoxid, Salicylsäure, Pyrithionderivate, Hexachlorophen oder quartäre Ammoniumverbindungen bzw. durch zellösende und entfettende Wirkstoffe wie beispielsweise Benzoylperoxid oder Teerextrakte lindern, jedoch nicht heilen.

Aus dem Stand der Technik sind also für die Glättung der Haut und Stärkung der Barrierefunktion, sei es aus kosmetischer oder medizinischer Sicht, sehr unterschiedliche Lösungen bekannt, die jeweils aber nur eine Teilaufgabe lösen und zudem noch mit starken Nebenwirkungen verbunden sein können. Die komplexe Aufgabe der Erfindung hat demnach darin bestanden, Wirkstoffe zur Verfügung zu stellen, die gleichzeitig gegen die Faltenbildung in der Haut (kosmetische Wirkung) und Hauterkrankungen (medizinische Wirkung) eingesetzt werden können und dabei eine hohe dermatologische und toxikologische Verträglichkeit besitzen.

JP-05-1314 offenbart eine Mischung aus Ginsengessenz und Cholesterolsulfat zur Vorbeugung gegen Faltenbildung.

### Beschreibung der Erfindung

Gegenstand der Erfindung ist die Verwendung von Slerolsulfaten als Wirkstoffe zur Herstellung von Mitteln zur Inhibierung von Serinproteasen, vorzugsweise von Elastasen.

Überraschenderweise wurde gefunden, daß Sterolsulfate Serinproteasen im allgemeinen und Elastasen im besonderen inaktivieren und so den Abbau von Makromolekülen wie Elastin, Kollagen etc. im Bindegewebsbereich verlangsamen. Auf diese Weise wird die altersbedingte Faltenbildung der Haut vermindert. Weiterhin wird durch topische Anwendung von Sterolsulfaten eine erhöhte Konzentration des Wirkstoffs im Bereich des Stratum corneums erzeugt und hierdurch ein erhöhter Zusammenhalt der Komeozyten und eine Verdickung der oberen Hautschichten bewirkt. Dieser Effekt ist besonders bei geschädigter Haut von Bedeutung, da hier das Stratum corneum seine Barrierefunktion nicht mehr erfüllen kann. Die Erfindung schließt weiterhin die Erkenntnis ein, daß Sterolsulfate durch die Elastaseinhibierung auch zur Vermeidung bzw. Verminderung von entzündlichen Reaktionen führt und so zur Behandlung von Hautkrankheiten dienen kann. Sterolsulfate, die in den oberen Schichten des Stratum comeums in Konzentrationen bis zu 4 Gew.-% vorkommen, sind schließlich als außerordentlich hautverträglich und toxikologisch unbedenklich bekannt.

### Sterolsulfate

Sterolsulfate stellen bekannte Stoffe dar, die beispielsweise durch Sulfatierung von Sterinen mit einem Komplex aus Schwefeltrioxid und Pyridin in Benzol hergestellt werden können [vgl. **J.Am.Chem.Soc. 63, 1259 (1941)**]. Unter Sterinen, die als Einsatzstoffe zur Herstellung der Sterolsulfate in Betracht kommen, sind solche Steroide zu verstehen, die nur am C-3 eine Hydroxylgruppe, sonst aber keine funktionellen Gruppen tragen. Es handelt sich also formal um Alkohole, weswegen diese Gruppe von Verbindungen auch gelegentlich als Sterole bezeichnet werden. In der Regel besitzen die Sterine 27 bis 30 Kohlenstoffatome und eine Doppelbindung in 5/6, gegebenenfalls 7/8, 8/9 oder anderen Positionen. Als Ausgangsstoffe kommen jedoch neben diesen ungesättigten Spezies auch die durch Härtung erhältlichen gesättigten Verbindungen in Frage. Typische Beispiele für geeignete Sterolsulfate sind solche auf Basis von Zoosterinen wie etwa tierisches Cholesterin, Lanosterinen aus Wollfett, Spongosterinen aus Schwämmen oder Stellasterinen aus Seesternen. Wegen der helleren Farbe der Sulfatierungsprodukte werden jedoch vorzugsweise Phytosterolsulfate eingesetzt, wie beispielsweise solche auf Basis von Ergosterinen, Campesterinen, Stigmasterinen und Sistosterinen. Die Sterolsulfate können als Alkali- und/oder Erdalkalimetallsalze, als Ammonium-, Alkylammonium-, Alkanolammoniumund/oder Glucammoniumsalze vorliegen. In der Regel werden sie in Form ihrer Natriumsalze eingesetzt.

Die Sterolsulfate können in reiner, fester Form in den Handel gebracht werden. Es ist jedoch ebenfalls möglich, sie als Rohstoffe für die Herstellung von kosmetischen oder pharmazeutischen Zubereitungen in Form von Lösungen in geeigneten Solventien wie beispielsweise Propylenglycol oder Butylenglycol oder als Compounds beispielsweise zusammen mit Fettalkoholethersulfaten anzubieten. In diesen konzentrierten Anbietungsformen kann der Gehalt an Sterolsulfaten im Bereich von 0,1 bis 10, vorzugsweise 1 bis 5 und insbesondere 2 bis 8 Gew.-% liegen.

### Gewerbliche Anwendbarkeit

Im Sinne der Erfindung können die Sterolsulfate als Wirkstoffe zur Herstellung von kosmetischen und/oder pharmazeutischen Zubereitungen eingesetzt werden. Typische Beispiele für solche Mittel sind Hautpflegemittel wie beispielsweise Antifaltencremes, Anticellulitiscremes oder Sonnenschutzlotionen sowie Salben zur Behandlung von Hauterkrankungen wie beispielsweise Cradle Cap, Schuppenflechte, Seborrhoische Dermatitis, Seborrhoe Sicca, Seborrhoe Oleosa, Psoriasis vulgaris, Ichtyosen oder UV-Erythemen. Üblicherweise können die Sterolsulfate in Mengen von 0,0001 bis 5, vorzugsweise 0,001 bis 1 und insbesondere 0,01 bis 0,1 Gew.-% - bezogen auf die Mittel - eingesetzt werden.

Die Zubereitungen können in untergeordneten Mengen mit den anderen Inhaltsstoffen kompatible anionische, nichtionische, kationische und/oder amphotere bzw. zwitterionische Tenside enthalten. Typische Beispiele für **anionische Tenside** sind Seifen, Alkylbenzolsulfonate, Alkansulfonate, Olefinsulfonate, Alkylethersulfonate, Glycerinethersulfonate, α-Methyl-estersulfonate, Sulfofettsäuren, Alkylsulfate, Fettalkoholethersulfate, Glycerinethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für **nichtionische Tenside** sind Fettalkoholpolyglycolether, Alkylphenolpolyglycolether, Fetlsäurepolyglycolester, Fettsäureamidpolyglycolether, Fettaminpolyglycolether, alkoxylierte Triglyceride, Mischether bzw. Mischformale, Alk(en)yloligoglykoside, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für **kationische Tenside** sind quartäre Ammoniumverbindungen und Esterquats, insbesondere quaternierte Fettsäuretrialkanolaminestersalze, Typische Beispiele für **amphotere bzw. zwitterionische Tenside** sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S. 54-124** oder **J.Falbe (ed.), "Katalysatoren, Tenside und Mineralöladditive", Thieme Verlag, Stuttgart, 1978, S. 123-217** verwiesen.

Femer können die Mittel als weitere Hilfs- und Zusatzstoffe Ölkörper, Emulgatoren, Überfettungsmittel, Stabilisatoren, Wachse, Konsistenzgeber, Verdickungsmittel, Kation-polymere, Siliconverbindungen, biogene Wirkstoffe, Antischuppenmittel, Filmbildner, Konservierungsmittel, Hydrotrope, Solubilisatoren, UV-Lichtschutzfilter, Farb- und Duftstoffe enthalten.

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₀-Fettsäuren mit linearen C₆-C₂₀-Fettalkoholen, Ester von verzweigten C₆-C₁₃-Carbonsäuren mit linearen C₆-C₂₀-Fettalkoholen, Ester von linearen C₆-C₁₈-Fettsäuren mit verzweigten Alkoholen, insbesondere 2-Ethylhexanol, Ester von linearen und/oder verzweigten Fettsäuren mit mehrwertigen Alkoholen (wie z.B. Dimerdiol oder Trimertriol) und/oder Guerbetalkoholen, Triglyceride auf Basis C₆-C₁₀-Fettsäuren, pflanzliche Öle, verzweigte primäre Alkohole, substituierte Cyclo-hexane, Guerbetcarbonate, Dialkylether, Siliconöle und/oder aliphatische bzw. naphthenische Kohlenwasserstoffe in Betracht.

Als **Emulgatoren** kommen beispielsweise nichtionogene Tenside aus mindestens einer der folgenden Gruppen in Frage:
- (b1): Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
- (b2): C_{12/18}-Fettsäuremono- und -diester von Anlagerungsprodukten von 1 bis 30 Mol Ethylenoxid an Glycerin;
- (b3): Glycerinmono- und -diester und Sorbitanmono- und -diester von gesättigten und ungesättigten Fettsäuren mit 6 bis 22 Kohlenstoffatomen und deren Ethylenoxid-anlagerungsprodukte;
- (b4): Alkylmono- und -oligoglycoside mit 8 bis 22 Kohlenstoffatomen im Alkylrest und deren ethoxylierte Analoga;
- (b5): Anlagerungsprodukte von 15 bis 60 Mol Ethylenoxid an Ricinusöl und/oder gehärtetes Ricinusöl;
- (b6): Polyol- und insbesondere Polyglycerinester wie z.B. Polyglycerinpolyricinoleat oder Polyglycerinpoly-12-hydroxystearat. Ebenfalls geeignet sind Gemische von Verbindungen aus mehreren dieser Substanzklassen;
- (b7): Anlagerungsprodukte von 2 bis 15 Mol Ethylenoxid an Ricinusöl und/oder gehärtetes Ricinusöl;
- (b8): Partialester auf Basis linearer, verzweigter, ungesättigter bzw. gesättigter C_{12/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit) sowie Polyglucoside (z.B. Cellulose);
- (b9): Trialkylphosphate;
- (b10): Wollwachsalkohole;
- (b11): Polysiloxan-Polyalkyl-Polyether-Copolymere bzw. entsprechende Derivate;
- (b12): Mischester aus Pentaerythrit, Fettsäuren, Citronensäure und Fettalkohol gemäß **DE-PS 11 65 574** sowie
- (b13): Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 20 24 051** als Rückfettungsmittel für kosmetische Zubereitungen bekannt.

C_{8/18}-Alkylmono- und oligoglycoside, ihre Herstellung und ihre Verwendung als oberflächenaktive Stoffe sind beispielsweise aus **US 3,839,318, US 3,707,535, US 3,547,828, DE-OS 19 43 689, DE-OS 20 36 472** und **DE-A1 30 01 064** sowie **EP-A 0 077 167** bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglyci-nate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquaternierte Difettsäuretriethanolaminester-Salze, besonders bevorzugt sind.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen. Als **Konsistenzgeber** kommen in erster Linie Fettalkohole mit 12 bis 22 und vorzugsweise 16 bis 18 Kohlenstoffatomen in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, kationische Stärke, Copolymere von Diallylammoniumsalzen und Acrylamiden, quaternierte VinylpyrrolidonNinylimidazol-Polymere wie z.B. Luviquat® (BASF AG, Ludwigshafen/ FRG), Kondensationsprodukte von Polyglycolen und Aminen, quaternierte Kollagenpolypeptide wie beispielsweise Lauryldimonium hydroxypropyl hydrolyzed Collagen (Lamequat®L, Grünau GmbH), quatemierte Weizenpolypeptide, Polyethylenimin, kationische Siliconpolymere wie z.B. Amidomethicone oder Dow Corning, Dow Corning Co./US, Copolymere der Adipinsäure und Dimethylaminohydroxypropyldiethylentrimamin (Cartaretine®, Sandoz/CH), Polyaminopolyamide wie z.B. beschrieben in der **FR-A 22 52 840** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen wie z.B. Dibrombutan mit Bisdialkylaminen wie z.B. Bis-Dimethylamino-1,3-propan, kationischer Guar-Gum wie z.B. Jaguar® CBS, Jaguar® C-17, Jaguar® C-16 der Celanese/US, quaternierte Ammoniumsalz-Polymere wie z.B. Mirapol® A-15, Mirapol® AD-1, Mirapol® A2-1 der Miranol/US.

Geeignete **Siliconverbindungen** sind beispielsweise Dimethylpolysiloxane, Methyl-phenylpolysiloxane, cyclische Silicone sowie amino-, fettsäure-, alkohol-, polyether-, epoxy-, fluor- und/oder alkylmodifizierte Siliconverbindungen, die bei Raumtemperatur sowohl flüssig als auch harzförmig vorliegen können. Typische Beispiele für **Fette** sind Glyceride, als **Wachse** kommen u.a. Bienenwachs, Paraffinwachs oder Mikrowachse gegebenenfalls in Kombination mit hydrophilen Wachsen, z.B. Cetylstearylalkohol in Frage. Als **Perlglanzwachse** können insbesondere Mono- und Difettsäureester von Polyalkylenglycolen, Partialglyceride oder Ester von Fettalkoholen mit mehrwertigen Carbonsäuren bzw. Hydroxycarbonsäuren verwendet werden. Als **Stabilisatoren** können Metallsalze von Fettsäuren wie z.B. Magnesium-, Aluminium- und/oder Zinkstearat eingesetzt werden. Unter **biogenen Wirkstoffen** sind beispielsweise Bisabolol, Allantoin, Phytantriol, Panthenol, AHA-Säuren, Pflanzenextrakte und Vitaminkomplexe zu verstehen. Als **Antischuppenmittel** können Climbazol, Octopirox und Zinkpyrethion eingesetzt werden. Gebräuchliche **Filmbildner** sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quatemäre Cellulose-Derivate, Kollagen, Hyaluronsäure bzw. deren Salze und ähnliche Verbindungen. Zur Verbesserung des Fließverhaltens können ferner **Hydrotrope** wie beispielsweise Ethanol, Isopropylalkohol, Propylenglycol oder Glucose eingesetzt werden. Als **Konservierungsmittel** eignen sich beispielsweise Phenoxyethanol, Formaldehydlösung, Parabene, Pentandiol oder Sorbinsäure.

Typische Beispiele für **UV-Lichtschutzfilter** sind 4-Aminobenzoesäure sowie ihre Ester und Derivate (z.B. 2-Ethylhexyl-p-dimethylaminobenzoat oder p-Dimethylaminobenzoesäureoctylester), Methoxyzimtsäure und ihre Derivate (z.B. 4-Methoxyzimtsäure-2-ethylhexylester), Benzophenone (z.B. Oxybenzon, 2-Hydroxy-4-methoxy-benzophenon), Dibenzoylmethane, Salicylatester, 2-Phenylbenzimadozol-5-sulfonsäure, 1-(4-tert.Butylphenyl)-3-(4'-methoxyphenyl)-propan-1,3-dion, 3-(4'-Methyl)benzylidenbornan-2-on, Methyl-benzylidencampher und dergleichen. Weiterhin geeignet sind auch feindisperse Metalloxide bzw. Salze, wie beispielsweise Titandioxid, Zinkoxid, Eisenoxid, Aluminiumoxid, Ceroxid, Zirkoniumoxid, Silicate (Talk) und Bariumsulfat. Schließlich kommen auch sekundäre Lichtschutzmittel vom Typ der Antioxidantien in Betracht, wie etwa Superoxid-Dismutase, Tocopherole (Vitamin E) und Ascorbinsäure (Vitamin C).

Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt- oder Heißprozesse erfolgen; vorzugsweise arbeitet man nach der Phaseninversionstemperatur-Methode.

### Beispiele

Die Elastase-Hemmwirkung wurde mit verschiedenen Sterolderivaten im Konzentrationsbereich zwischen 2,5 und 1000 ppm untersucht. Die Ergebnisse sind in Tabelle 1 zusammengefaßt. Die Beispiele 1 und 2 sind erfindungsgemäß, die Beispiele V1 bis V3 dienen zum Vergleich.

**Tabelle 1**

| **Elastasehemmwirkung [%-rel] bei verschiedenen Konzentrationen (2,5 bis 1000 ppm)** | | | | | | |
|---|---|---|---|---|---|---|
| **Bsp.** | **Inhibitor** | **2,5 ppm** | **5 ppm** | **12,5 ppm** | **500 ppm** | **1000 ppm** |
| 1 | Cholesterolsulfat-Natriumsalz | 49 | 92 | 100 | - | - |
| 2 | Phytosterolsulfat-Natriumsalz | 31 | 64 | 98 | - | - |
| V1 | Cholesterolacetat | 0 | 0 | 0 | 33 | 59 |
| V2 | Pytosterolsuccinat | 0 | 0 | 0 | 0 | 0 |
| V3 | Pytosterolphosphat | 0 | 0 | 0 | 0 | 30 |

## Patentansprüche

1. Verwendung von Sterolsulfaten zur Herstellung von Mitteln zur Behandlung von Hauterkrankungen wie z.B. Cradle Cap, Schuppenflechte, Seborrhoische Dermatitis, Seborrhoe Sicca, Seborrhoe Oleosa, Psoriasis vulgaris, Ichtyosen oder UV-Erythemen durch Inhibierung von Serinproteasen.

2. Verwendung von Sterolsulfaten zur Inhibierung von Serinproteasen in kosmetischen Mitteln.

3. Verwendung nach Ansprüchen 1 oder 2 **dadurch gekennzeichnet, daß** die Sterolsulfate auf Basis von Phytosterinen, Cholesterin und/oder deren Härtungsprodukten eingesetzt werden.

4. Verwendung nach Ansprüchen 1 bis 3 **dadurch gekennzeichnet, daß** die Sterolsulfate in den Mitteln in Mengen von 0,0001 bis 5 Gew.% - bezogen auf die Mittel - eingesetzt werden.

## Claims

1. The use of sterol sulfates for the production of preparations for the treatment of skin diseases such as, for example, cradle cap, psoriasis, seborrhoeic dermatitis, seborrhoea sicca, seborrhoea oleosa, psoriasis vulgaris, ichtyoses or UV erythemas by inhibiting serine proteases.

2. The use of sterol sulfates for inhibiting serine proteases in cosmetic preparations.

3. The use claimed in claim 1 or 2, **characterized in that** the sterol sulfates are based on phytosterols, cholesterol and/or hydrogenation products thereof.

4. The use claimed in claims 1 to 3, **characterized in that** the sterol sulfates are used in the preparations in quantities of 0.0001 to 5% by weight, based on the particular preparation.

## Revendications

1. Utilisation de sulfates de stérols pour la préparation de produits destinés au traitement de maladies de peau, comme par exemple le casque séborrhéique, le parapsoriasis, la dermatite séborrhéique, la séborrhée sèche, la séborrhée huileuse, le psoriasis vulgaire, les ichtyoses ou les érythèmes dus aux UV, par inhibition des sérine protéases.

2. Utilisation de sulfates de stérols pour l'inhibition de sérine protéases dans des produits cosmétiques.

3. Utilisation selon la revendication 1 ou 2,
**caractérisée en ce qu'**
on utilise les sulfates de stérols à base de phytostérols, cholestérol et/ou leurs produits d'hydrogénation.

4. Utilisation selon les revendications 1 à 3,
**caractérisée en ce que**
les sulfates de stérols sont utilisés dans les produits en quantités de 0,0001 à 5 % en poids, par rapport aux produits.
